# EUROPÄISCHE PATENTANMELDUNG

(11) **EP 3 130 318 A2**
(43) Veröffentlichungstag der Anmeldung: **15.02.2017**
(21) Anmeldenummer: 16183731.5
(22) Anmeldetag: 11.08.2016
(51) Int. Cl.: A61F 2/58, A61F 2/50, B25J 15/08, A61B 34/30

(54) **GREIFWERKZEUG UND GREIFVORRICHTUNG**

(30) Priorität: 11.08.2015 DE 202015005616 U
(71) Anmelder: Hannecke, Wolf-Dietrich, 37154 Northeim (DE)
(72) Erfinder: Hannecke, Wolf-Dietrich, 37154 Northeim (DE)
(74) Vertreter: Hofstetter, Schurack & Partner

(57) **Zusammenfassung**

Die Erfindung betrifft ein Greifwerkzeug (1) zum Greifen eines Gegenstands (14), welches als 3D-Druckbauteil ausgebildet ist, und ein erstes zumindest bereichsweise nicht-geradliniges Greifwerkzeugteil (2) und ein zweites zumindest bereichsweise nicht-geradliniges Greifwerkzeugteil (3) aufweist, wobei die beiden Greifwerkzeugteile (2, 3) jeweils einen endseitigen Greifbereich (4, 5, 6, 7) aufweisen und die Greifwerkzeugteile (2, 3) mit einem nicht-geradlinigen Filmscharnier (8) derart bewegbar miteinander verbunden sind, dass bei einer durch eine Scharnierbewegung des Filmscharniers (8) hervorgerufenen Relativbewegung (11) der Greifwerkzeugteile (2, 3) zueinander ein Aufeinanderzubewegen (12) oder ein Voneinanderwegbewegen (13) von den zum Greifen eines Gegenstands (14) vorgesehenen endseitigen Greifbereichen (4, 5, 6, 7) der Greifwerkzeugteile (2, 3) auftritt.

## Beschreibung

Die Erfindung betrifft ein Greifwerkzeug zum Greifen eines Gegenstands. Zudem betrifft die Erfindung eine Greifvorrichtung mit einem Greifwerkzeug.

Greifwerkzeuge zum Greifen von Gegenständen sind aus dem Stand der Technik bekannt. So kann ein Greifwerkzeug beispielsweise als eine durch eine menschliche Hand bedienbare Greifzange ausgebildet sein. Weiterhin kann ein Greifwerkzeug beispielsweise bei einem industriellen Produktionsablauf eingesetzt werden. So kann das Greifwerkzeug beispielsweise an einen Roboter angebracht werden, um dadurch einen Gegenstand greifen zu können. Der mit dem Greifwerkzeug gegriffene Gegenstand kann dann beispielsweise von einer Position in eine andere gebracht werden oder zum Bearbeiten gehalten werden. Diese Greifwerkzeuge sind sehr schwer und unhandlich.

Es ist Aufgabe der vorliegenden Erfindung, ein Greifwerkzeug sowie eine Greifvorrichtung zu schaffen, welches beziehungsweise welche bei hoher Funktionalität mit geringerem Gewicht ausgebildet ist.

Diese Aufgabe wird durch ein Greifwerkzeug sowie eine Greifvorrichtung gemäß den Merkmalen nach Anspruch 1 gelöst.

Ein erfindungsgemäßes Greifwerkzeug ist zum Greifen eines Gegenstands ausgebildet. Das Greifwerkzeug ist, insbesondere als Ganzes, als 3D-Druckmodell ausgebildet. Das Greifwerkzeug weist ein erstes, zumindest bereichsweise nicht-geradliniges Greifwerkzeugteil und ein zweites, zumindest bereichsweise nicht-geradliniges Greifwerkzeug auf. Die beiden Greifwerkzeugteile weisen jeweils einen endseitigen Greifbereich auf und sind mit einem nicht-geradlinigen Filmscharnier miteinander verbunden. Die Greifwerkzeugteile sind derart miteinander verbunden, dass bei einer durch eine Scharnierbewegung des Filmscharniers hervorgerufenen Relativbewegung der Greifwerkzeugteile zueinander ein Aufeinanderzubewegen oder ein Voneinanderwegbewegen von den zum Greifen eines Gegenstands vorgesehenen endseitigen Greifbereichen der Greifwerkzeugteile auftritt.

Durch die zwei nicht-geradlinigen Greifwerkzeugteile mit dem nichtgeradlinigen Filmscharnier kann das Greifwerkzeug mit weniger Arbeitsaufwand hergestellt werden und mit geringerem Gewicht ausgebildet sein. So ist das Greifwerkzeug insbesondere lediglich durch das nichtgeradlinige Filmscharnier bewegbar und nicht durch sonstige Lager und/oder Gelenke. Das nicht-geradlinige Filmscharnier hat den weiteren Vorteil, dass es spielfrei ist. Dadurch kann das Greifwerkzeug präziser betrieben werden.

Durch die Ausbildung des Greifwerkzeugs als 3D-Druckbauteil kann es leichtgewichtiger als bekannte Greifwerkzeuge hergestellt werden. Dennoch kann es auch mit einer komplexen Geometrie formpräzise ausgebildet werden. Bei 3D-Druckverfahren werden dreidimensionale Werkstücke schichtweise aufgebaut. Der Aufbau erfolgt computergesteuert aus einem oder mehreren flüssigen oder festen Werkstoffen nach vorgegebenen Maßen und Formen. Beim Aufbau finden physikalische oder chemische Härtungs- oder Schmelzprozesse statt. Vorzugsweise wird als Werkstoff für das 3D-Druckverfahren ein Kunststoff oder ein Kunstharz gewählt. Das Erzeugen des 3D-Druckbauteils mit dem 3D-Druckverfahren wird üblicherweise als generatives beziehungsweise additives Fertigungsverfahren beschrieben. Vorzugsweise ist es vorgesehen, dass das 3D-Druckbauteil mit einem Stereolithographieverfahren, einem DLP-Verfahren (DLP - digital light processing), einem Polyjet-Modelling-Verfahren oder einem Fused-Deposition-Modelling-Verfahren erzeugt wird. Vorteilhaft ist dies jeweils, weil das Greifwerkzeugteil dadurch schnell und mit geringem Arbeitsaufwand erzeugt werden kann.

Die Funktion des Greifwerkzeugs wird durch die nicht-geradlinigen Greifwerkzeugteile und dem nicht-geradlinigen Filmscharnier bereitgestellt. So wird die Relativbewegung der Greifwerkzeugteile zueinander aufgrund der Scharnierbewegung des Filmscharniers hervorgerufen. Durch die Relativbewegung können die Greifwerkzeugteile aufeinander zu bewegt oder voneinander weg bewegt werden. So kann der Gegenstand durch das Aufeinanderzubewegen gegriffen werden und durch das Voneinanderwegbewegen losgelassen werden. Insbesondere ist dazu keine separate Bewegung der endseitigen Greifbereiche notwendig. Die endseitigen Greifbereiche werden mitsamt den Greifwerkzeugteilen bewegt.

Insbesondere ist es vorgesehen, dass das Filmscharnier über seine gesamte Länge nicht-geradlinig ausgebildet ist. Dadurch wird die Relativbewegung der beiden Greifwerkzeugteile ebenfalls über die gesamte Länge der Greifwerkzeugteile hervorgerufen. Das Greifen des Gegenstands kann dadurch präziser und insbesondere auch bezüglich der Greifkraft sehr feindosiert erfolgen.

Weiterhin ist es insbesondere vorgesehen, dass das erste Greifwerkzeugteil und/oder das zweite Greifwerkzeugteil sichelförmig ausgebildet sind. Durch die sichelförmige Ausgestaltung wird das Greifen des Gegenstands vereinfacht, da insbesondere der Bewegungsablauf der Greifwerkzeugteile sehr kontinuierlich und somit ruckfrei und ohne Sprungbewegungen der Greifwerkzeugteile durchgeführt werden kann. So wird das Aufeinanderzubewegen und das Voneinanderwegbewegen der Greifwerkzeugteile vereinfacht und auch der zum Greifen des Gegenstands vorgesehene endseitige Greifbereich des jeweiligen Greifwerkzeugteils wird dadurch entsprechend schmal zum sehr zielgerichteten Greifen des Gegenstands bereitgestellt.

Weiterhin ist es insbesondere vorgesehen, dass durch die eckenfreie, nichtgeradlinige Ausgestaltung der Greifwerkzeugteile und das eckenfreie, nichtgeradlinige Filmscharnier ein mechanisches Wirkprinzip ausgebildet ist, bei welchem das Greifwerkzeug ausgehend von einer den Gegenstand greifenden Greifstellung automatisch, insbesondere fremdkraftlos, in eine nicht greifende Grundstellung überführt ist. So ist das Greifwerkzeug also derart ausgebildet, dass durch eine Eigenspannung der Greifwerkzeugteile eine Rückführung von der Greifstellung in die nichtgreifende Grundstellung durchgeführt werden kann, ohne den Einfluss einer fremden Kraft von außerhalb des Greifwerkzeugs zu benötigen. Vorteilhaft ist dies, weil dadurch keine Kraft zum Freigeben des Gegenstands in der nicht greifenden Grundstellung nötig ist. Wird der Gegenstand also beispielsweise durch das Greifwerkzeug gegriffen und das Greifwerkzeug befindet sich in der Greifstellung, so kann durch Entfernen der momentanen Krafteinwirkung auf das Greifwerkzeug automatisch von der Greifstellung in die nicht greifende Grundstellung gewechselt werden. Der Gegenstand wird dadurch also von dem Greifwerkzeug energiesparender, da diese Bewegung nicht zwingend mit einem motorischen Antrieb erfolgen muss, und verschleißärmer freigegeben beziehungsweise losgelassen.

Vorzugsweise ist es vorgesehen, dass das erste Greifwerkzeugteil und/oder das zweite Greifwerkzeugteil zu einem jeweiligen Greifbereich des jeweiligen Greifwerkzeugteils hin verjüngt zulaufend ausgebildet ist. Durch das verjüngte Zulaufen des jeweiligen Greifwerkzeugteils kann der Greifbereich schmäler und für ein präziseres Greifen bereitgestellt werden. So kann der Greifbereich beispielsweise situationsbedingt hinsichtlich seiner Abmessungen angepasst werden. So ist ein kleinerer Greifbereich sinnvoll, um kleinere Gegenstände zu greifen, während ein größerer Greifbereich sinnvoll ist, um größere Gegenstände mit einer größeren Oberfläche sicherer zu greifen. Insbesondere ist dadurch ein Greifwerkzeugteil an seinem freien Ende größenminimiert ausgebildet, so dass auch das Gewicht dort relativ klein gehalten ist. Hebelkräfte werden dadurch an diesen Greifwerkzeugteilen verkleinert und die Bewegung von großen Massen von Greifwerkzeugkomponenten des Greifwerkzeugs vermieden.

In einer weiteren Ausführungsform ist es vorgesehen, dass die Greifwerkzeugteile Seitenränder aufweisen, die in ihren Höhenrichtungen jeweils mit einer oberen und einer unteren Begrenzung begrenzt sind, wobei das Filmscharnier an einer oberen Begrenzung oder an einer unteren Begrenzung eines jeweiligen Seitenrands des ersten Greifwerkzeugteils und/oder des zweiten Greifwerkzeugteils mündet. Durch die Mündung des Filmscharniers an die obere Begrenzung oder an die untere Begrenzung kann das Greifwerkzeug aufwandsärmer und unkomplizierter hergestellt werden. Das Filmscharnier kann beispielsweise auch hinsichtlich einer bevorzugten Richtung der Relativbewegung an die untere Begrenzung oder an die obere Begrenzung münden. Vorteilhaft ist, dass das Greifwerkzeug dadurch vielfältig und situationsabhängig bereitgestellt werden kann.

In einer weiteren Ausführungsform kann es vorgesehen sein, dass die Greifwerkzeugteile Seitenränder aufweisen, die in ihren Höhenrichtungen jeweils mit einer oberen und einer unteren Begrenzung begrenzt sind, wobei das Filmscharnier beabstandet zu einer oberen Begrenzung oder zu einer unteren Begrenzung des jeweiligen Seitenrands des ersten Greifwerkzeugteils und/oder des zweiten Greifwerkzeugteils mündet. Durch das Münden des Filmscharniers an den Seitenrand des jeweiligen Greifwerkzeugteils mit dem Abstand zu der oberen Begrenzung und der unteren Begrenzung wird das Filmscharnier also beispielsweise mittig an dem Seitenrand angeordnet. So kann das Filmscharnier beispielsweise für eine Relativbewegung in beide Richtungen ausgebildet sein. Vorteilhaft ist also wiederum die Variantenvielfalt bei der Herstellung des Greifwerkzeugs.

Weiterhin kann es vorgesehen sein, dass die Greifwerkzeugteile Seitenränder aufweisen, die in ihren Höhenrichtungen jeweils mit einer oberen und einer unteren Begrenzung begrenzt sind, wobei das Filmscharnier an die Seitenränder mündende Enden aufweist, die aufgeweitet ausgebildet sind, insbesondere genau auf die gesamte Höhe der Seitenränder aufgeweitet sind. So erhöht sich also die Materialstärke des Filmscharniers in Richtung des Seitenrands wegen der Enden. Dadurch kann das Filmscharnier und somit das Greifwerkzeug robuster gegenüber Verschleiß bereitgestellt werden. Das Filmscharnier kann also weniger leicht von dem jeweiligen Seitenrand abbrechen, wenn dieses mit dem aufgeweiteten, Ende an das jeweilige Greifwerkzeugteil angebunden ist.

In einer weiteren Ausführungsform kann es vorgesehen sein, dass das erste Greifwerkzeugteil und/oder das zweite Greifwerkzeugteil an dem endseitigen Greifbereich des ersten Greifwerkzeugteils und/oder dem endseitigen Greifbereich des zweiten Greifwerkzeugteils mit einer Aufnahmemulde ausgebildet sind. Die Aufnahmemulde kann beispielsweise als Einschnitt in den jeweiligen endseitigen Greifbereich ausgebildet sein. Durch die Aufnahmemulde wird das Greifen des Gegenstands einfacher und zuverlässiger ermöglicht. So kann die Aufnahmemulde derart ausgebildet sein, dass der Gegenstand bezüglich seiner Form passend in die Aufnahmemulde aufgenommen wird. Das Greifen des Gegenstands erfolgt also durch die Aufnahmemulde zuverlässiger. Die Sicherheit des Greifwerkzeugs kann dadurch erhöht werden.

In einer weiteren Ausführungsform ist es vorzugsweise vorgesehen, dass an dem endseitigen Greifbereich des ersten Greifwerkzeugteils und/oder dem endseitigen Greifbereich des zweiten Greifwerkzeugs ein zylinderförmiges Endstück angeordnet ist. Das zylinderförmige Endstück kann beispielsweise elastisch ausgebildet sein. So kann das zylinderförmige Endstück beispielsweise als Fingerkuppe bezeichnet werden. Das zylinderförmige Endstück kann beispielsweise direkt an dem jeweiligen Greifwerkzeugteil befestigt sein oder aber indirekt über eine in dem Greifwerkzeug angeordnete skelettartige Halteeinheit befestigt sein. So kann beispielsweise eine Schnur oder ein Draht oder ein Seil verlaufen, wodurch die zylinderförmigen Endstücke miteinander verbunden sind und dadurch an dem Greifwerkzeug gehalten werden. Durch die Zylinderförmigen Endstücke kann das Greifwerkzeug feinfühliger nach dem Gegenstand greifen. Auch kann beispielsweise ein Verkratzen des Gegenstands durch die zylinderförmigen Endstücke verhindert werden. Das zylinderförmige Endstück kann beispielsweise aus einem Elastomer gefertigt sein.

Vorzugsweise ist es vorgesehen, dass das erste Greifwerkzeugteil mit einem zweiten, nicht-geradlinigen Filmscharnier mit einem dritten, nichtgeradlinigen Greifwerkzeugteil relativ bewegbar verbunden ist und das zweite Greifwerkzeugteil mit einem dritten, nicht-geradlinigen Filmscharnier mit einem vierten, nicht-geradlinigen Greifwerkzeugteil relativ bewegbar verbunden ist. Durch das dritte Greifwerkzeugteil und das vierte Greifwerkzeugteil wird das Greifwerkzeug stabiler ausgebildet. So kann das Greifwerkzeug dadurch beispielsweise eine größere Kraft auf den Gegenstand in der Greifstellung ausüben. Es können dadurch beispielsweise schwerere Gegenstände gegriffen werden. Gleichzeitig können die Gegenstände auch zuverlässiger, also fester, gegriffen werden. Das dritte Greifwerkzeugteil und das vierte Greifwerkzeugteil werden insbesondere spiegelverkehrt zu dem ersten Greifwerkzeugteil und dem zweiten Greifwerkzeugteil angeordnet. Vorzugsweise werden das erste Greifwerkzeugteil, das zweite Greifwerkzeugteil, das dritte Greifwerkzeugteil und das vierte Greifwerkzeugteil als ganzes 3D-Druckbauteil erzeugt. Dadurch kann der Arbeitsaufwand bei der Erzeugung reduziert werden und das Greifwerkzeug kann mit einer stärker ausgeprägten Greiffähigkeit bereitgestellt werden.

In einer weiteren Ausführungsform ist es vorzugsweise vorgesehen, dass das dritte Greifwerkzeugteil und das vierte Greifwerkzeugteil mit einem vierten, nicht-geradlinigen Filmscharnier verbunden sind. Dadurch können das dritte Greifwerkzeugteil und das vierte Greifwerkzeugteil also nicht nur mit dem ersten Greifwerkzeugteil und dem zweiten Greifwerkzeugteil, sondern auch untereinander verbunden sein. Hierdurch wird das Greifwerkzeug insgesamt wiederum robuster ausgebildet. Der Gegenstand kann also wiederum fester und zuverlässiger gegriffen werden.

Vorzugsweise ist es vorgesehen, dass das erste Greifwerkzeugteil, das zweite Greifwerkzeugteil, das dritte Greifwerkzeugteil und das vierte Greifwerkzeugteil eine im Querschnitt über die Filmscharniere verformbare Raute bilden. Die Raute entsteht beispielsweise durch das Verbinden der Greifwerkzeugteile mit den Filmscharnieren. Durch die rautenförmige Ausgestaltung des Greifwerkzeugs kann beispielsweise das zumindest eine zylinderförmige Endstück bezüglich einer in der Greifstellung wirkenden Kraftverteilung ausgeglichen aufgenommen werden. So kann die Kraft, welche durch den Gegenstand auf der einen Seite des zylinderförmigen Endstücks vorhanden ist, auf der anderen Seite, welche dem jeweiligen Greifwerkzeugteil zugewandt ist, durch die Greifwerkzeugteile insgesamt und gleichmäßig verteilt aufgenommen werden. Weiterhin ist durch den rautenförmigen Querschnitt des Greifwerkzeugs ein Verdrehen des Greifwerkzeugs während der Greifstellung erschwert.

Die Erfindung betrifft auch eine Greifvorrichtung mit zumindest einem erfindungsgemäßen Greifwerkzeug. Die Greifvorrichtung kann auch mehrere erfindungsgemäße Greifwerkzeuge umfassen.

Vorzugsweise ist es vorgesehen, dass die Greifvorrichtung als chirurgisches Instrument ausgebildet ist. Durch das chirurgische Instrument können beispielsweise Operationen durchgeführt werden. Zu den chirurgischen Instrumenten zählen alle medizinischen Instrumente. So kann das Greifwerkzeug beispielsweise an einem Roboterarm, welcher von der Greifvorrichtung umfasst wird, für das chirurgische Instrument bereitgestellt werden.

In einer weiteren Ausführungsform kann es vorgesehen sein, dass die Greifvorrichtung als medizinische Prothese ausgebildet ist. Eine Prothese bezeichnet den Ersatz von Gliedmaßen, Organen oder Organteilen durch künstlich geschaffene, funktionell ähnliche Produkte. So kann die Greifvorrichtung beispielsweise als Beinprothese, Armprothese oder Handprothese ausgebildet sein. Eine Exoprothese kann in diesem Fall mit weniger Arbeitsaufwand und mit geringem Gewicht bereitgestellt werden.

In einer weiteren Ausbildungsform kann es vorgesehen sein, dass die Greifvorrichtung als Greifzangenwerkzeug ausgebildet ist. So kann das Greifzangenwerkzeug beispielsweise an einen Roboter oder einen Roboterarm angeordnet sein. Das Greifzangenwerkzeug kann dann beispielsweise in einer Produktionsanlage zur Fertigung eingesetzt werden. Auch kann das Greifzangenwerkzeug in diesem Zusammenhang zur Automatisierung eingesetzt werden. Vorteilhaft ist wiederum eine einfachere und solidere Ausgestaltung der Greifvorrichtung durch das erfindungsgemäße Greifwerkzeug.

Ausführungsbeispiele der Erfindung werden nachfolgend anhand schematischer Zeichnungen näher erläutert. Es zeigen:
- Fig. 1: eine schematische Darstellung eines Greifwerkzeugs mit einem ersten Greifwerkzeugteil, einem zweiten Greifwerkzeugteil und einem nicht-geradlinigen Filmscharnier in einem entfalteten Zustand;
- Fig. 2: eine schematische Darstellung des Greifwerkzeugs mit dem Filmscharnier in gefaltetem Zustand;
- Fig. 3: eine schematische Schnittdarstellung des Greifwerkzeugs, bei welcher das Filmscharnier an einer unteren Begrenzung eines Seitenrands des jeweiligen Greifwerkzeugteils mündet;
- Fig. 4: eine schematische Schnittdarstellung des Greifwerkzeugs, bei welchem das Filmscharnier beabstandet zu einer oberen Begrenzung und einer unteren Begrenzung des Seitenrands des jeweiligen Greifwerkzeugteils mündet;
- Fig. 5: eine schematische Schnittdarstellung des Greifwerkzeugs analog zu Fig. 3, wobei an die Seitenränder Enden münden, die aufgeweitet ausgebildet sind;
- Fig. 6: eine schematische Schnittdarstellung des Greifwerkzeugs analog zu Fig. 4, wobei die an die Seitenränder mündenden Enden, aufgeweitet ausgebildet sind;
- Fig.7: eine schematische Schnittdarstellung des Greifwerkzeugs analog zu Fig. 5, wobei die an die Seitenränder mündenden Enden nicht-linear aufgeweitet ausgebildet sind;
- Fig.8: eine schematische Schnittdarstellung des Greifwerkzeugs analog zu Fig. 6, wobei die an die Seitenränder mündenden Enden nicht-linear aufgeweitet ausgebildet sind;
- Fig.9: eine perspektivische Darstellung eines Ausführungsbeispiels des Greifwerkzeugs mit dem ersten Greifwerkzeugteil, dem zweiten Greifwerkzeugteil, einem dritten Greifwerkzeugteil und einem vierten Greifwerkzeugteil; und
- Fig. 10: eine perspektivische Darstellung eines weiteren Ausführungsbeispiels des Greifwerkzeugs, bei welchem an dem endseitigen Greifbereich des jeweiligen Greifwerkzeugteils ein zylinderförmiges Endstück angeordnet ist.

In den Figuren werden gleiche oder funktionsgleiche Elemente mit den gleichen Bezugszeichen versehen.

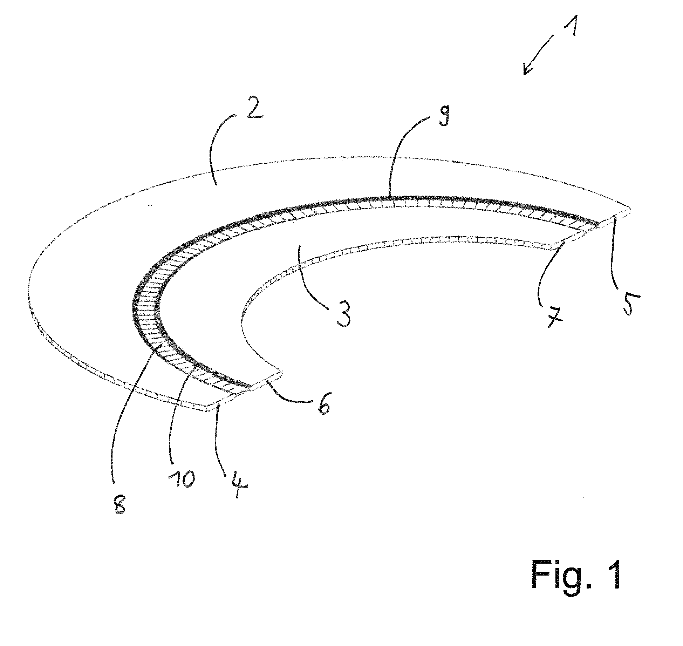

In Fig. 1 ist ein Greifwerkzeug 1 mit einem ersten, nicht-geradlinigen Greifwerkzeugteil 2 und einem zweiten, nicht-geradlinigen Greifwerkzeugteil 3 gezeigt. Das erste Greifwerkzeugteil 2 weist einen ersten endseitigen Greifbereich 4 und einen zweiten endseitigen Greifbereich 5 auf. Das zweite Greifwerkzeugteil 3 weist einen ersten Greifbereich 6 und einen zweiten Greifbereich 7 auf. Weiterhin sind das erste Greifwerkzeugteil 2 und das zweite Greifwerkzeugteil 3 mit einem nicht-geradlinigen ersten Filmscharnier 8 miteinander verbunden.

Gemäß Fig. 1 ist das erste Filmscharnier 8 in einem entfalteten Zustand gezeigt. In dem entfalteten Zustand greift das Greifwerkzeug vorzugsweise nicht. Weiterhin sind das erste Greifwerkzeugteil und das zweite Greifwerkzeugteil 3 gemäß dem Ausführungsbeispiel sichelförmig ausgebildet. Zudem ist das erste Filmscharnier 8 über seine gesamte Länge nicht-geradlinig ausgebildet.

Das Greifwerkzeug 1 ist gemäß dem Ausführungsbeispiel als Ganzes als 3D-Druckbauteil ausgebildet. Das 3D-Druckbauteil wird mit einem 3D-Druckverfahren erzeugt. Durch das 3D-Druckverfahren wird ein generatives beziehungsweise additives Fertigungsverfahren beschrieben. Vorliegend wird das 3D-Druckverfahren vorzugsweise durch eine Stereolithographie, ein digital light processing, ein Polyjet-Modelling oder ein fused deposition modelling beschrieben. Als Werkstoff sind insbesondere Kunststoffe oder Kunstharze vorgesehen.

Weiterhin weist das erste Greifwerkzeugteil 2 einen Seitenrand 9 auf und das zweite Greifwerkzeugteil 3 weist einen Seitenrand 10 auf.

Das erste Greifwerkzeugteil 2 und das zweite Greifwerkzeugteil 3 sind zu dem jeweiligen Greifbereich 4, 5, 6, 7 hin verjüngt zulaufend ausgebildet.

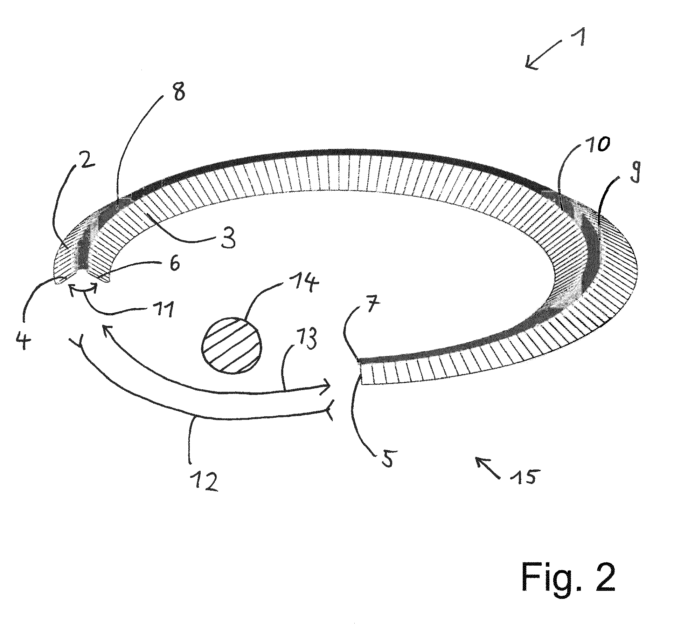

Fig. 2 zeigt das Greifwerkzeugteil 1 mit dem ersten Filmscharnier 8 in einem gefalteten Zustand. Durch das erste Filmscharnier 8 sind die Greifwerkzeugteile 2, 3 derart bewegbar miteinander verbunden, dass bei einer Scharnierbewegung des ersten Filmscharniers 8 eine Relativbewegung 11 der Greifwerkzeugteile 2, 3 hervorgerufen. Durch die Relativbewegung 11 tritt ein Aufeinanderzubewegen 12 oder ein Voneinanderwegbewegen 13 der Greifbereiche 4, 5, 6, 7 auf. So bewegen sich der erste Greifbereich 4 des ersten Greifwerkzeugteils 2 und der erste Greifbereich 6 des zweiten Greifwerkzeugteils 3 auf den zweiten Greifbereich 5 des ersten Greifwerkzeugteils 2 und den zweiten Greifbereich 7 des zweiten Greifwerkzeugteils 3 zu oder voneinander weg. Das Aufeinanderzubewegen 12 tritt auf, wenn das erste Filmscharnier 8 zunehmend stärker gefaltet wird. Das Voneinanderwegbewegen 13 tritt auf, wenn das erste Filmscharnier 8 zunehmend weiter entfaltet wird, bis es beispielsweise in dem entfalteten Zustand gemäß Fig. 1 vorliegt.

Weiterhin zeigt Fig. 2 einen Gegenstand 14, welcher durch das Greifwerkzeug 1 gegriffen werden kann. So wird der Gegenstand 14 bei dem Aufeinanderzubewegen 12 mit den Greifbereichen 4, 5, 6, 7 der Greifwerkzeugteile 2, 3 gegriffen. So ist für das Greifen lediglich die Relativbewegung 11 in Richtung des gefalteten ersten Filmscharniers 8 notwendig.

Ergänzend oder alternativ ist das Greifwerkzeug 1 ausgehend von einer den Gegenstand 14 greifenden - nicht weiter dargestellten - Greifstellung automatisch in eine nicht greifende Grundstellung 15 überführbar. Die nicht greifende Grundstellung 15 ist gemäß Fig. 2 lediglich beispielhaft gezeigt und kann in vielfältigen anderen Stellungen der Greifwerkzeugteile 2, 3 zueinander vorliegen, insbesondere jedoch so, dass der Gegenstand 14 noch nicht gegriffen wird. Das automatische Überführen von der greifenden Greifstellung in die nicht greifende Grundstellung 15 wird durch das mechanische Wirkprinzip ermöglicht, welches insbesondere durch eine Materialspannung des ersten Greifwerkzeugteils 2 und/oder des zweiten Greifwerkzeugteils 3 und/oder des ersten Filmscharniers 8 ausgebildet ist. So erfolgt das Überführen von der greifenden Greifstellung in die nicht greifende Grundstellung 15 vorzugsweise ohne die Einwirkung von einer fremden Kraft, welche separat von dem Greifwerkzeug 1 erzeugt wird. Lediglich das Aufeinanderzubewegen 12 und die dazu korrespondierende Relativbewegung 11, welche den greifenden Zustand beziehungsweise die greifende Greifstellung auslöst, wird insbesondere durch Fremdkrafteinwirkung ausgeführt.

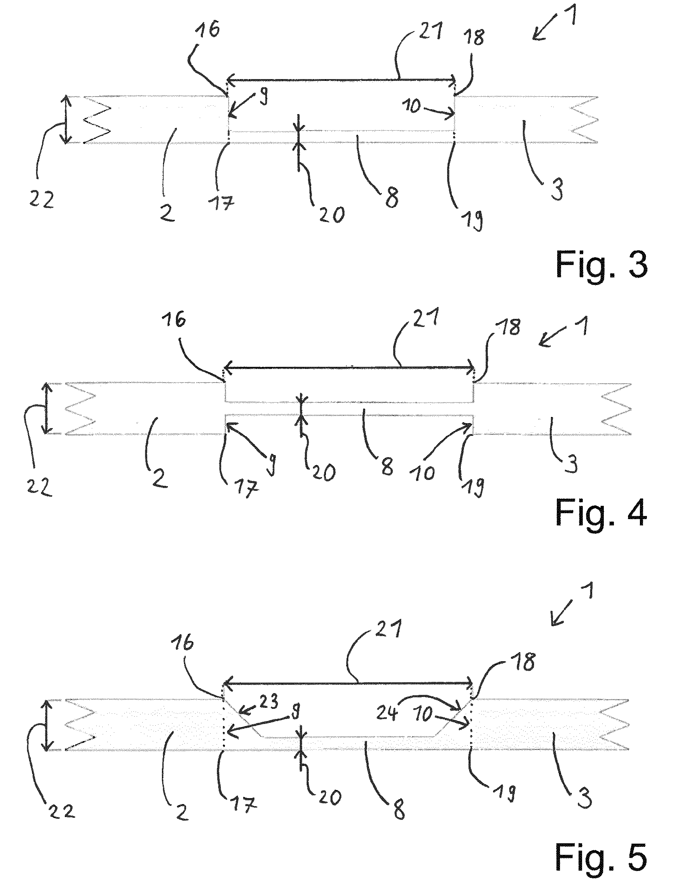

Fig. 3 zeigt das Greifwerkzeug 1 in einer Schnittdarstellung. Der Seitenrand 9 des ersten Greifwerkzeugteils 2 weist eine obere Begrenzung 16 und eine untere Begrenzung 17 auf. Der Seitenrand 10 des zweiten Greifwerkzeugteils 3 weist eine obere Begrenzung 18 und eine untere Begrenzung 19 auf. Gemäß dem Ausführungsbeispiel von Fig. 3 mündet das erste Filmscharnier 8 an der unteren Begrenzung 17 des Seitenrands 9 und an der unteren Begrenzung 19 des Seitenrands 10. Das erste Filmscharnier 8 kann sich beispielsweise in Höhenrichtung mit einer Höhe 20 erstrecken. Weiterhin kann sich das erste Filmscharnier 8 über eine Breite 21 in Breitenrichtung erstrecken. Die Höhe 20 kann beispielsweise bis zu 0,5 mm oder mehr betragen, während die Breite 21 beispielsweise bis zu 10 mm oder mehr betragen kann. Eine Greifwerkzeugteilhöhe 22 in Höhenrichtung des ersten Greifwerkzeugteils 2 und/oder des zweiten Greifwerkzeugteils 3 kann beispielsweise bis zu 2 mm oder mehr betragen.

Fig. 4 zeigt das Greifwerkzeug 1 ebenfalls in einer Schnittdarstellung. Gemäß diesem Ausführungsbeispiel mündet das erste Filmscharnier 8 beabstandet zu der oberen Begrenzung 16, 18 und der unteren Begrenzung 17, 19 an den jeweiligen Seitenrand 9, 10.

Fig. 5 zeigt das Greifwerkzeug 1 in einer weiteren Schnittdarstellung. Gemäß diesem Ausführungsbeispiel mündet das erste Filmscharnier 8 an der unteren Begrenzung 17, 19 des jeweiligen Seitenrands 9, 10. Das erste Filmscharnier 8 weist ein erstes, an den zweiten Rand 9 mündendes Ende 23 und ein zweites an den Seitenrand 10 mündendes Ende 24 auf. Die Enden 23, 24 weiten sich auf die gesamte Höhe der Seitenränder 9, 10 auf, also bis zu der oberen Begrenzung 16, 18 des jeweiligen Seitenrands 9, 10.

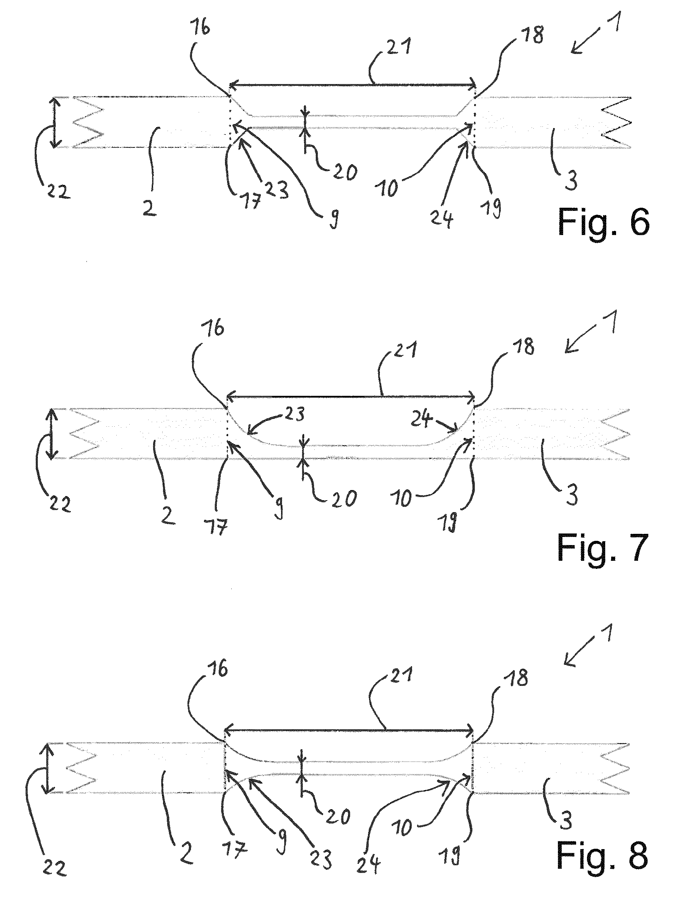

Fig. 6 zeigt eine weitere Schnittdarstellung des Greifwerkzeugs 1. Gemäß diesem Ausführungsbeispiel sind die Enden 23, 24 auf die gesamte Höhe der Seitenränder 9, 10 aufgeweitet, und das erste Filmscharnier 8 ist beabstandet zu der oberen Begrenzung 16, 17 und der unteren Begrenzung 17, 19 des jeweiligen Seitenrands 9, 10 angeordnet.

Fig. 7 zeigt eine weitere Schnittdarstellung des Greifwerkzeugs 1. Das erste Filmscharnier 8 mündet an der unteren Begrenzung 17, 19 des jeweiligen Seitenrands 9, 10. Die Enden 23, 24 weiten sich nicht-linear, beispielsweise parabelförmig, auf.

Fig. 8 zeigt eine weitere Schnittdarstellung des Greifwerkzeugs 1 analog zu Fig. 7. Jedoch ist gemäß dem Ausführungsbeispiel von Fig. 8 ein Münden des ersten Filmscharniers 8 beabstandet zu der oberen Begrenzung 16, 18 und der unteren Begrenzung 17, 19 an den jeweiligen Seitenrand 9, 10 vorgesehen.

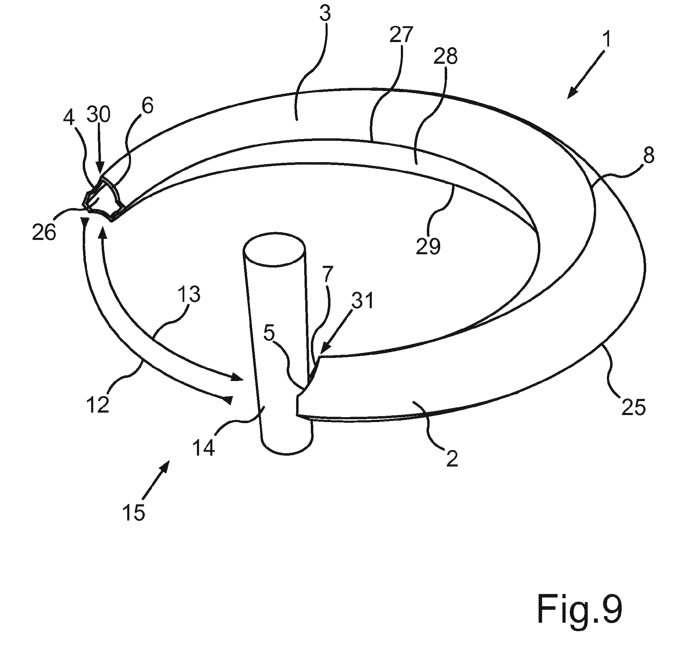

Fig. 9 zeigt das Greifwerkzeug 1 zusammen mit dem Gegenstand 14. Gemäß diesem Ausführungsbeispiel ist das erste Greifwerkzeugteil 2 durch ein nicht-geradliniges zweites Filmscharnier 25 mit einem nicht-geradlinigen dritten Greifwerkzeugteil 26 relativ bewegbar verbunden. Weiterhin ist das zweite Greifwerkzeugteil 3 mit einem nicht-geradlinigen dritten Filmscharnier 27 und mit einem nicht-geradlinigen vierten Greifwerkzeugteil 28 relativ bewegbar verbunden. Darüber hinaus sind das dritte Greifwerkzeugteil 26 und das vierte Greifwerkzeug 28 mit einem nicht-geradlinigen vierten Filmscharnier 29 verbunden. Somit bilden das erste Greifwerkzeugteil 2, das zweite Greifwerkzeug 3, das dritte Greifwerkzeugteil 26 und das vierte Greifwerkzeugteil 28 eine im Querschnitt über die Filmscharniere 8, 25, 27, 29 verformbare Raute.

Die Beschreibung des ersten Filmscharniers 8 kann auch für das zweite Filmscharnier 25, das dritte Filmscharnier 27 und/oder das vierte Filmscharnier 29 herangezogen werden. Ebenso kann die Beschreibung des ersten Greifwerkzeugteils 2 und/oder des zweiten Greifwerkzeugteils 3 auch für das dritte Greifwerkzeugteil 26 und/oder das vierte Greifwerkzeugteil 28 herangezogen werden.

Das Greifwerkzeug 1 gemäß Fig. 9 weist an den ersten Greifbereichen 4, 6 eine erste Aufnahmemulde 30 und an den zweiten Greifbereichen 5, 7 eine zweite Aufnahmemulde 31 auf. Durch die erste Aufnahmemulde 30 und/oder die zweite Aufnahmemulde 31 kann der Gegenstand 14 beispielsweise besser beziehungsweise fester gegriffen werden. So kann die Aufnahmemulde 30, 31 derart ausgebildet sein, dass der Gegenstand 14 davon zumindest teilweise umgeben wird. Die Aufnahmemulde 30, 31 kann beispielsweise als Einschnitt in das jeweilige Greifwerkzeugteil 2, 3, 26, 28 ausgebildet sein.

Gemäß dem Ausführungsbeispiel von Fig. 9 liegt das Greifwerkzeug 1 ebenfalls beispielsweise in der nicht-greifenden Grundstellung 15 vor. So ist der Gegenstand 14 lediglich in Kontakt mit den zweiten Greifbereichen 5, 7.

Durch das Aufeinanderzubewegen 12 der Greifbereiche 4, 5, 6, 7 wird der Gegenstand 14 dann gegriffen.

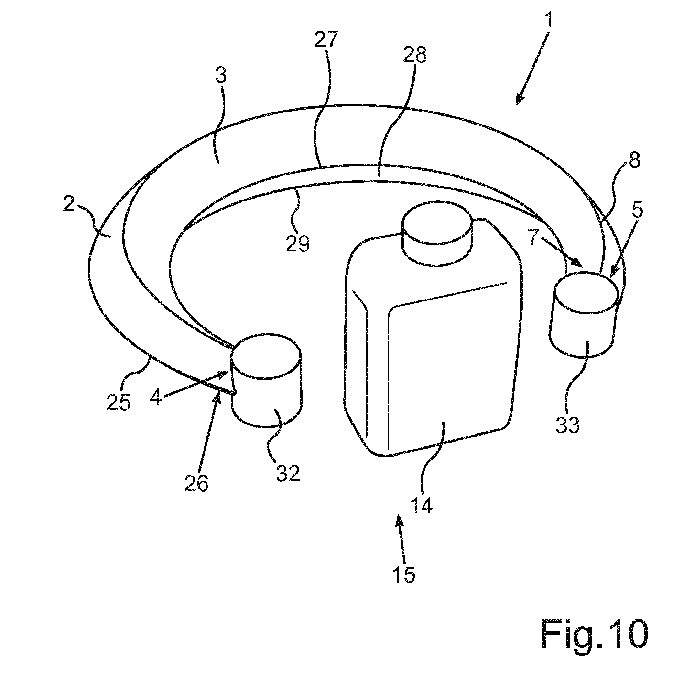

Fig. 10 zeigt das Greifwerkzeug 1 analog zu Fig. 9. An den ersten Greifbereichen 4, 6 ist ein erstes zylinderförmiges Endstück 32 und an den zweiten Greifbereichen 5, 7 ist ein zweites zylinderförmiges Endstück 33 angeordnet. Das erste Endstück 32 und/oder das zweite Endstück 33 können beispielsweise aus einem Elastomer gefertigt sein. Die Endstücke 32, 33 sind also vorzugsweise elastisch ausgebildet und können beispielsweise beim Greifen einen Schutz vor Beschädigung für den Gegenstand 14 bereitstellen. So kann der Gegenstand 14 mit den Endstücken 32, 33 feinfühliger gegriffen werden.

Das Greifwerkzeug 1 kann beispielsweise als chirurgisches Instrument oder als medizinische Prothese oder als Greifzangenwerkzeug ausgebildet sein. So kann das Greifwerkzeug 1 beispielsweise in einer Produktionsanlage zur automatisierten Fertigung eingesetzt werden. Das Greifwerkzeug 1 kann beispielsweise mit einem Roboterarm verbunden werden, um den Gegenstand 14 zu greifen.

## Patentansprüche

1. Greifwerkzeug (1) zum Greifen eines Gegenstands (14), welches als 3D-Druckbauteil ausgebildet ist, und ein erstes zumindest bereichsweise nichtgeradliniges Greifwerkzeugteil (2) und ein zweites zumindest bereichsweise nicht-geradliniges Greifwerkzeugteil (3) aufweist, wobei die beiden Greifwerkzeugteile (2, 3) jeweils zumindest einen endseitigen Greifbereich (4, 5, 6, 7) aufweisen und die Greifwerkzeugteile (2, 3) mit einem nichtgeradlinigen Filmscharnier (8) derart bewegbar miteinander verbunden sind, dass bei einer durch eine Scharnierbewegung des Filmscharniers hervorgerufenen Relativbewegung (11) der Greifwerkzeugteile (2, 3) zueinander ein Aufeinanderzubewegen (12) oder ein Voneinanderwegbewegen (13) von den zum Greifen eines Gegenstands (14) vorgesehenen endseitigen Greifbereichen (4, 5, 6, 7) der Greifwerkzeugteile (2, 3) auftritt.

2. Greifwerkzeug (1) nach Anspruch 1, **dadurch gekennzeichnet, dass** das Filmscharnier (8) über seine gesamte Länge nicht-gradlinig ausgebildet ist.

3. Greifwerkzeug (1) nach Anspruch 1 oder 2, **dadurch gekennzeichnet, dass** das erste Greifwerkzeugteil (2) und/oder das zweite Greifwerkzeugteil (3) sichelförmig ausgebildet ist.

4. Greifwerkzeug (1) nach einem der vorhergehenden Ansprüche, **dadurch gekennzeichnet, dass** durch die eckenfreie nicht-geradlinige Ausgestaltung der Greifwerkzeugteile (2, 3) und das eckenfreie nicht-geradlinige Filmscharnier (8) ein mechanisches Wirkprinzip ausgebildet ist, bei welchem das Greifwerkzeug (1) ausgehend von einer den Gegenstand (14) greifenden Greifstellung automatisch in eine nicht-greifende Grundstellung (15) überführbar ist.

5. Greifwerkzeug (1) nach einem der vorhergehenden Ansprüche, **dadurch gekennzeichnet, dass** das erste Greifwerkzeugteil (2) und/oder das zweite Greifwerkzeugteil (3) zu einem jeweiligen Greifbereich (4, 5, 6, 7) des jeweiligen Greifwerkzeugteils (2, 3) hin verjüngt zulaufend ausgebildet ist.

6. Greifwerkzeug (1) nach einem der vorhergehenden Ansprüche, **dadurch gekennzeichnet, dass** die Greifwerkzeugteile (2, 3) Seitenränder (9, 10) aufweisen, die in ihren Höhenrichtungen jeweils mit einer oberen Begrenzung (16, 18) und einer unteren Begrenzung (17, 19) begrenzt sind, wobei das Filmscharnier (8) an einer oberen Begrenzung (16, 18) oder an einer unteren Begrenzung (17, 19) eines jeweiligen Seitenrands (9, 10) des ersten Greifwerkzeugteils (2) und/oder des zweiten Greifwerkzeugteils (3) mündet.

7. Greifwerkzeug (1) nach einem der Ansprüche 1 bis 5, **dadurch gekennzeichnet, dass** die Greifwerkzeugteile (2, 3) Seitenränder (9, 10) aufweisen, die in ihren Höhenrichtungen jeweils mit einer oberen Begrenzung (16, 18) und einer unteren Begrenzung (17, 19) begrenzt sind, wobei das Filmscharnier (8) beabstandet zu einer oberen Begrenzung (16, 18) oder zu einer unteren Begrenzung (17, 19) eines jeweiligen Seitenrands (9, 10) des ersten Greifwerkzeugteils (2) und/oder des zweiten Greifwerkzeugteils (3) mündet.

8. Greifwerkzeug (1) nach einem der vorhergehenden Ansprüche, **dadurch gekennzeichnet, dass** die Greifwerkzeugteile (2, 3) Seitenränder (9, 10) aufweisen, die in ihren Höhenrichtungen jeweils mit einer oberen Begrenzung (16, 18) und einer unteren Begrenzung (17, 19) begrenzt sind, wobei das Filmscharnier (8) an die Seitenränder (9, 10) mündende Enden (23, 24) aufweist, die aufgeweitet ausgebildet sind, insbesondere genau auf die gesamte Höhe (22) der Seitenränder (9, 10) aufgeweitet sind.

9. Greifwerkzeug (1) nach einem der vorhergehenden Ansprüche, **dadurch gekennzeichnet, dass** das erste Greifwerkzeugteil (2) und/oder das zweite Greifwerkzeugteil (3) an dem endseitigen Greifbereich (4, 5) des ersten Greifwerkzeugteils (2) und/oder dem endseitigen Greifbereich (6, 7) des zweiten Greifwerkzeugteils (3) mit einer Aufnahmemulde (30, 31) ausgebildet ist.

10. Greifwerkzeug (1) nach einem der vorhergehenden Ansprüche, **dadurch gekennzeichnet, dass** an dem endseitigen Greifbereich (4, 5) des ersten Greifwerkzeugteils (2) und/oder dem endseitigen Greifbereich (6, 7) des zweiten Greifwerkzeugteils (3) ein zylinderförmiges Endstück (32, 33) angeordnet ist.

11. Greifwerkzeug (1) nach einem der vorhergehenden Ansprüche, **dadurch gekennzeichnet, dass** das erste Greifwerkzeugteil (2) mit einem zweiten nicht-geradlinigen Filmscharnier (25) mit einem dritten nicht-geradlinigen Greifwerkzeugteil (26) relativ bewegbar verbunden ist und das zweite Greifwerkzeugteil (3) mit einem dritten nicht-geradlinigen Filmscharnier (27) mit einem vierten nicht-geradlinigen Greifwerkzeugteil (28) relativ bewegbar verbunden ist.

12. Greifwerkzeug (1) nach Anspruch 11, **dadurch gekennzeichnet, dass** das dritte Greifwerkzeugteil (26) und das vierte Greifwerkzeugteil (28) mit einem vierten nicht-geradlinigen Filmscharnier (29) verbunden sind.

13. Greifwerkzeug (1) nach Anspruch 11 oder 12, **dadurch gekennzeichnet, dass** das erste Greifwerkzeugteil (2), das zweite Greifwerkzeugteil (3), das dritte Greifwerkzeugteil (26) und das vierte Greifwerkzeugteil (28) eine im Querschnitt über die Filmscharniere (8, 25, 27, 29) verformbare Raute bilden.

14. Greifvorrichtung mit zumindest einem Greifwerkzeug (1) nach einem der vorhergehenden Ansprüche.

15. Greifvorrichtung nach Anspruch 14,
**dadurch gekennzeichnet, dass**
die Greifvorrichtung als chirurgisches Instrument ausgebildet ist, oder als medizinische Prothese ausgebildet ist, oder als Greifzangenwerkzeug ausgebildet ist.
